# EUROPÄISCHE PATENTSCHRIFT

(11) **EP 1 012 228 B1**
(45) Veröffentlichungstag und Bekanntmachung des Hinweises auf die Patenterteilung: **24.04.2002**
(21) Anmeldenummer: 99929238.6
(22) Anmeldetag: 15.06.1999
(51) Int. Cl.: C12M 1/32, B01L 3/00

(54) **GERÄT ZUR AUFNAHME UND ÜBERTRAGUNG BIOLOGISCHER PROBEN**
DEVICE FOR TAKING UP AND TRANSFERRING BIOLOGICAL SAMPLES
APPAREIL POUR COLLECTER ET TRANSFERER DES ECHANTILLONS BIOLOGIQUES

(30) Priorität: 15.06.1998 DE 19826244
(43) Veröffentlichungstag der Anmeldung: 28.06.2000
(73) Patentinhaber: Gesellschaft für Biotechnologische Forschung mbH (GBF), 38124 Braunschweig (DE)
(72) Erfinder: BLOECKER, Helmut, D-38124 Braunschweig (DE); KAUER, Gerhard, D-38124 Braunschweig (DE); NEELEN, Bernhard, D-38124 Braunschweig (DE); SCHOEN, Oliver, D-38124 Braunschweig (DE)
(74) Vertreter: Boeters, Hans Dietrich, Dr.
(86) Internationale Anmeldenummer: EP9904161
(87) Internationale Veröffentlichungsnummer: WO9966023

(56) Entgegenhaltungen:
- WO-A-87/05323
- WO-A-87/07911
- CH-A- 433 603
- US-A- 4 115 200
- US-A- 4 235 971
- US-A- 4 659 673

## Beschreibung

Die Erfindung betrifft ein Gerät gemäß Gattungsbegriff des Patentanspruchs 1.

Ein derartiges Gerät ist aus US-A-4,235,971 in einer Form bekannt, bei der nadelartige Probenaufnahmeelemente in dem Träger in Reihen und Spalten angeordnet sind und der Träger automatisch gesteuert auf den betreffenden Kulturboden absenkbar, sodann wieder anhebbar, über das Probenaufnahmebehältnis verfahrbar und dort erneut absenkbar und wieder anhebbar ist, um schließlich, ggf. nach Durchfahren weiterer Positionen, in eine Ausgangslage zurückzukehren. Ein solches und ähnliche Geräte erleichtern erheblich das ansonsten häufig noch manuell erfolgende Auflesen und Übertragen der Proben in beispielsweise 384 Kammern einer Mikrotiterplatte, wie auch Fehler infolge Unachtsamkeiten ausgeschlossen werden. Indessen sind derartige automatische Geräte aufwendig in Anschaffung und Unterhalt. Außerdem erfordert die Maßvorgabe von Mikrotiter-platten eine Systemanpassung.

Hiervon ausgehend liegt der Erfindung die Aufgabe zugrunde, ein Gerät zur gezielten Aufnahme biologischer Proben und deren Übertragung in vorbestimmte Positionen, wie z.B. diejenigen der Kammern einer Mikrotiterplatte, zu schaffen, das einerseits gegenüber einem entsprechenden manuellen Arbeitsablauf eine erhebliche Arbeits- und Zeitersparnis bei erhöhter Arbeitssicherheit mit sich bringt, andererseits aber einen wesentlich geringeren Aufwand erfordert als die betreffenden bekannten vollautomatischen Geräte.

Diese Aufgabe ist erfindungsgemäß durch ein Gerät mit den Merkmalen des Patentanspruchs 1 gelöst. Die Unteransprüche geben darüber hinausgehend vorteilhafte Ausgestaltungsmöglichkeiten an.

Erfahrungsgemäß läßt sich mit dem betreffenden Gerät bereits nach kurzer Einarbeitungszeit gegenüber der herkömmlichen manuellen Verfahrensweise eine erhebliche Zeitersparnis wie auch Arbeitssicherheit im Sinne der Vermeidung von Fehlern wie etwa in Form von Kontaminationen erzielen. Dabei ist das betreffende Gerät verhältnismäßig einfach und entsprechend robust, wie auch eine Systemanpassung auf verhältnismäßig einfache Weise möglich ist.

Ein flexibles Band, in dem nadelartige Probenaufnahmeelemente nebeneinanderliegend in gleichmäßigen Teilungsabständen angeordnet sind, in Verbindung mit einer Schalt- und Führungsvorrichtung, die geeignet ist das Band entsprechend den betreffenden Teilungsabständen schrittweise fortzuschalten und in einer Weise umzulenken, daß in jeder Schrittschaltstellung des Bandes jeweils eines der Probenaufnahmeelemente aus der Reihe der übrigen Probenaufnahmeelemente hervortritt, ist zwar andererseits bereits aus WO 87/05323 bekannt. Dabei handelt es sich aber um ein automatisch betriebenes Gerät mit zwei gleichartigen, zur Probenaufnahme bzw. -abgabe bestimmten, automatisch auf- und abbewegten Schalt- und Führungsvorrichtungen, von deren erster das Band automatisch zu der zweiten weitergeführt wird. Die Probenabgabe erfolgt jeweils an der gleichen Stelle in jeweils einen für die Aufnahme einer einzigen Probe bestimmten Behälter. Mithin wird die Anordnung der Probenaufnahmeelemente auf dem Band in bestimmten Teilungsabständen nicht für eine Probenabgabe in vorbestimmten unterschiedlichen Positionen, wie etwa denen einzelner Kammern einer Mikrotiterplatte, ausgenutzt.

Schließlich ist noch aus US-A-4,659,673 ein manuell bedienbares Probenübertragungsgerät mit in bestimmten Teilungsabständen angeordneten nadelartigen Probenaufnahmeelementen bekannt, bei dem es sich allerdings um eine Art Stempel handelt, in dem die Probenaufnahmeelemente in großer Zahl zweidimensional angeordnet sind. Für eine gezielte Aufnahme einzelner Proben und deren Übertragung an bestimmte, registrierbare Stellen ist ein solches Gerät ungeeignet.

Nachfolgend wird ein bevorzugtes Ausführungsbeispiel anhand der Figuren genauer beschrieben. Von diesen zeigt
- Fig. 1: das betreffende Gerät bei Anwendung zur Probeaufnahme von einem Kulturboden,
- Fig. 2: die gleichzeitige Abgabe einer Vielzahl vorausgehend aufgenommener Proben in einer Kammerreihe einer entsprechenden Mikrotiterplatte mit Hilfe einer Komponente des Geräts aus Fig. 1 in Gestalt eines kammer-artigen flexiblen Bandes,
- Fig. 3: ein Gerät ähnlich demjenigen aus Fig. 1, teilweise aufgeschnitten,
- Fig. 4: die wesentlichsten darin enthaltenen Teile und
- Fig. 5: eine Einzelansicht des erwähnten kammartigen flexiblen Bandes.

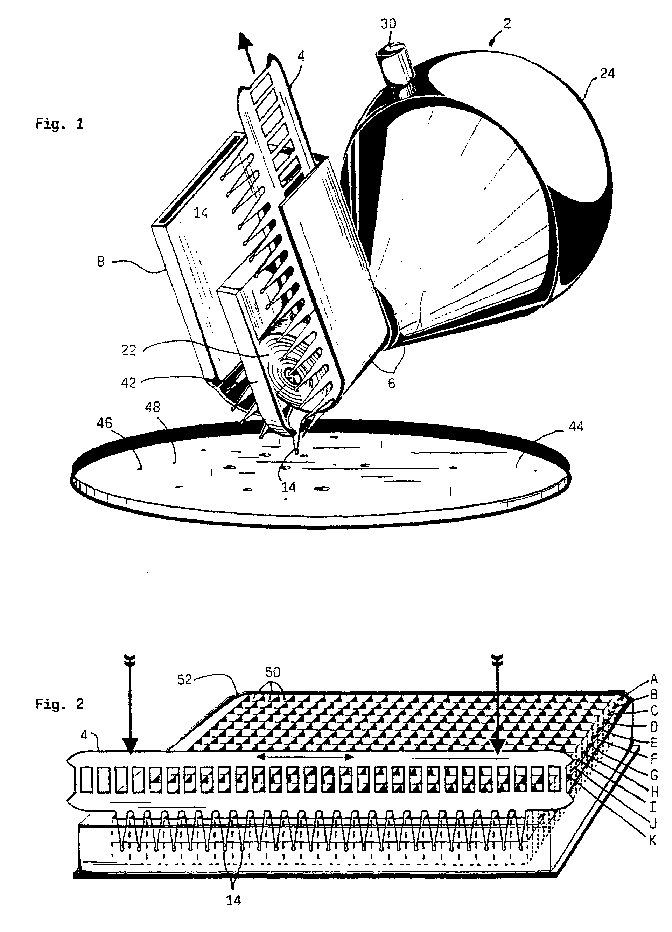

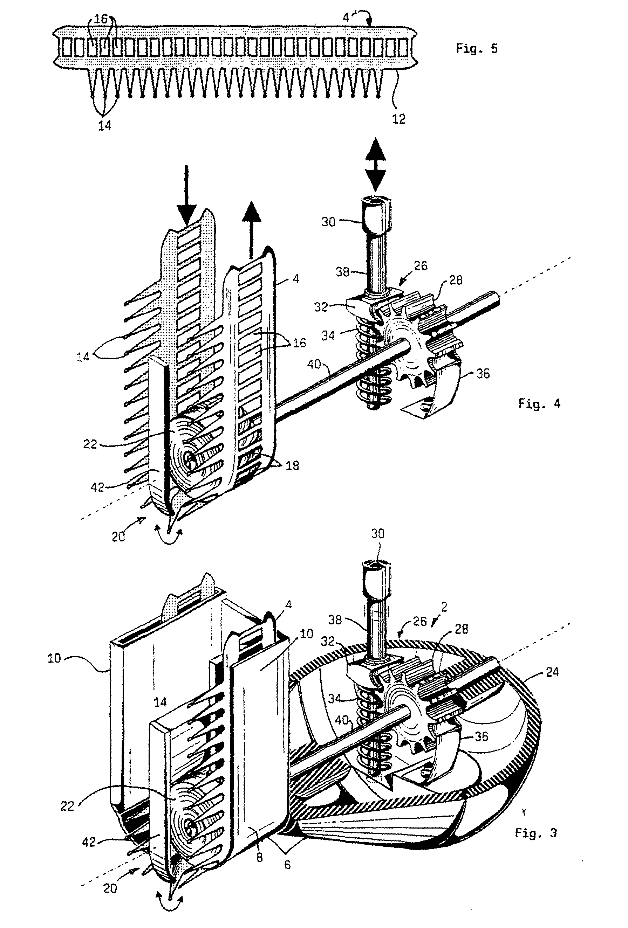

Das in Fig. 1 im Ganzen und in Fig. 3 teilweise gezeigte Gerät 2 besteht im wesentlichen aus einem kammartigen Band 4, bei-spielsweise aus V2A-Stahl, sowie einer handhaltbaren Schalt- und Führungsvorrichtung 6 für das Band 4, aus der dieses willkürlich entnehmbar ist. Genauer gesagt ist das Band 4 in einer U-förmigen Führung 8 der Schalt- und Führungsvorrichtung 6 geführt, deren beide Schenkel 10 stirnseitig offenstehen.

Wie am besten in Fig. 5 erkennbar, besteht das Band 4 aus einem flachen, leiterartigen Abschnitt 12, aus dem nadelartige Probenaufnahmeelemente 14 in gleichmäßigen Teilungsabständen seitlich hervortreten. Der leiterartige Abschnitt 12 besitzt in Form der Aussparungen 16 zwischen seinen "Leitersprossen" eine Transportperforation, in welche Zähne 18 eines im Scheitelbereich 20 der Führung 8 angeordneten Vorschubzahnrades 22 der Schalt- und Führungsvorrichtung 6 einzugreifen vermögen. Der Teilungsabstand der Probenaufnahmeelemente 14 kann, muß jedoch nicht, demjenigen der Aussparungen 16 des Bandes 4 entsprechen.

Indessen weist die Schalt- und Führungsvorrichtung 6 innerhalb eines an die Führung 8 anschließenden Gehäuses 24 ein Schrittschaltwerk 26 im wesentlichen bestehend aus einem Schaltzahnrad 28, einem Druckknopf 30, einer Schaltklaue 32, einer Rückholfeder 34 in Gestalt einer Wendeldruckfeder sowie eine Sperrklinke 36 in Gestalt einer Blattfeder auf. Der Druckknopf 30 tritt zu seiner manuellen Betätigung aus dem Gehäuse 24 hervor. Die Schaltklaue 32 ist auf einem zylindrischen Schaft 38 des Druck-knopfes 30 kippfähig gelagert und sucht unter der Einwirkung der Rückholfeder 34 mit dem Schaltzahnrad 28 in Eingriff zu treten, ebenso wie die Sperrklinke 36 bestrebt ist, in das Zahnrad 28 einzugreifen. Wird der Druckknopf 30 niedergedrückt, so führt die Schaltklaue 32 das Zahnrad 28 unter Überwindung einer Rastung seitens der Sperrklinke 36 um eine Zahnteilung mit. Wird der Druckknopf 30 daraufhin freigegeben, so kehrt er mitsamt der Schaltklaue 32 unter der Einwirkung der Rückholfeder 34 in seine Ausgangsstellung zurück, wobei ein weiterer Eingriff der Schaltklaue 32 mit dem Zahnrad 28 aufgrund ihrer Kippfähigkeit gegenüber dem Schaft 38 unterbleibt, während die Sperrklinke 36 eine Rückwärtsdrehung des Zahnrads 28 verhindert. Auf diese Weise kann das Zahnrad 28 durch wiederholte Betätigung (Niederdrücken) des Druckknopfs 30 um jeweils eine Zahnteilung im gleichen Sinn gedreht werden.

Das Zahnrad 28 ist wie auch das Vortriebszahnrad 22 fest auf einer in dem Gehäuse 24 gelagerten Welle 40 gelagert, so daß sich Drehungen des Zahnrads 28 dem Vorschubzahnrad 22 und damit auch dem in der Führung 8 befindlichen Band 4 mitteilen.

Die Führung 8 ist in ihrem Scheitelbereich 20 und an dem in Vorschubrichtung des Bandes daran anschließenden Schenkel 10 nach der dem Gehäuse 24 abgewandten Seite hin offen und läßt so die nadelartigen Probenaufnahemeelemente 14 des darin befindlichen Bandes 4 hervortreten. Wie im einzelnen aus den Figuren 1, 3 und 4 ersichtlich, ist im Scheitelbereich 20 der Führung 8 eine Gleitkufe 42 fest angeordnet, durch die jeweils ein gerade in der Symmetrieebene der Führung 8 liegendes Probenaufnahmeelement 14 eine Auslenkung radial von der Achse der Welle 40 hinweg nach außen erfährt. Mit diesem jeweils einzigen nach außen ausgelenkten Probenaufnahmeelement 14 lassen sich, wie aus Fig. 1 ersichtlich, von einem Kulturboden 44, beispielsweise der Oberfläche einer Agarschale, weg Proben,wie z.B. 46, 48, aufnehmen, eine jede mit einem anderen Probenaufnahmeelement 14. Dazu ist das Band 4 in der angegebenen Weise durch Betätigung des Druckknopfes 30 jeweils um eine Teilung weiterschaltbar, so daß jeweils ein neues Probenaufnahmeelement 14 durch die Gleitkufe 42 ausgelenkt wird.

Sind auf diese Weise sämtliche Proben aufgenommen, so kann das betreffende Band 4 nach einigen Leerschaltschritten mittels des Druckknopfes 30 aus der Führung 8 entnommen werden. Dann kann das so mit Proben beladene Band 4 gemäß Fig. 2, mit beiden Händen in gestreckter Form gehalten, mit seinen Probenaufnahmeelementen 14 in eine Reihe der Kammern 50 einer dazu passenden Mikrotiterplatte 52 eingeführt werden, um die einzelnen Proben in die einzelnen Kammern 50 dieser Reihe zu übertragen. In herkömmlicher Weise sind die Kammern 50 gewöhnlich mit einem pufferhaltigen Material gefüllt, welches mit der anschließend jeweils anzustellenden Analyse verträglich ist. Nach Reinigung kann das betreffende Band 4 erneut in die Führung 8 eingesetzt und zu einer erneuten Probenaufnahme und -übertragung mittels des Gerätes 2 verwendet werden. Indessen sollten zur Ermöglichung eines möglichst kontinuierlichen Arbeitsablaufs zumindest so viele Bänder 4 in Vorrat gehalten werden, wie die verwendeten Mikrotiterplaten 52 Reihen von Kammern 50 aufweisen.

## Patentansprüche

1. Gerät (2) zur gezielten Aufnahme biologischer Proben (z.B. 46, 48) und deren Übertragung in in einer Reihe liegende, gleiche Teilungsabstände aufweisende Positionen eines flachen Probenaufnahmebehältnisses mittels einer entsprechenden Anzahl nadelartiger Probenaufnahmeelemente (14) innerhalb eines gesteuert beweglichen Trägers, in dem diese Probenaufnahmeelemente in gleichmäßigen Teilungsabständen entsprechend denjenigen der betreffenden Positionen in dem Probenaufnahmebehältnis angeordnet sind, **dadurch gekennzeichnet, daß** der Träger aus einem flexiblen Band (4) besteht, in dem die Probenaufnahmeelemente (14) nach Art der Zinken eines Kammes nebeneinanderliegend mit Teilungsabständen entsprechend denjenigen der betreffenden Positionen in dem Probenaufnahmebehältnis angeordnet sind, und daß das Gerät (2) des weiteren eine manuell bedienbare, handhaltbare Schalt- und Führungsvorrichtung (6) für das Band (4) aufweist, die geeignet ist, das Band entnehmbar aufzunehmen, sowie entsprechend den Teilungsabständen der Probenaufnahmeelemente in dem Band schrittweise fortzuschalten und in einer Weise umzulenken, daß in jeder Schrittschaltstellung des Bandes jeweils eines der Probenaufnahmeelemente (14) mit seiner Spitze aus der Reihe der übrigen Probenaufnahmeelemente nach außen hervortritt.

2. Gerät (2) nach Anspruch 1, **dadurch *gekennzeichnet,* daß** die Probenaufnahmeelemente (14) seitlich an dem Band (4) innerhalb der gleichen Ebene ausgebildet sind.

3. Gerät (2) nach Anspruch 1 oder 2, **dadurch *gekennzeichnet,* daß** das Band (4) eine Transportperforation (16) und die Schaltund Führungsvorrichtung (6) ein dazu passendes Vorschubzahnrad (22) aufweist.

4. Gerät (2) nach Anspruch 2 und 3, **dadurch *gekennzeichnet*, daß** das Band (4) aus Metallfolie, vorzugsweise V2A-Folie, und daraus vorzugsweise durch elektrolytische Ätzung, hergestellt ist.

5. Gerät (2) nach Anspruch 3, **dadurch *gekennzeichnet,* daß** das Vorschubzahnrad (22) durch ein handbetätigbares Schrittschaltwerk (26) um jeweils einen Teilungswinkel entsprechend dem Teilungsabstand der Probenaufnahmeelemente (14) in dem Band (4) fortschaltbar ist.

6. Gerät (2) nach einem der vorhergehenden Ansprüche, **dadurch *gekennzeichnet,* daß** die Schalt- und Führungsvorrichtung (6) eine etwa U-förmige Führung (8) für das Band (4) aufweist, in deren Scheitelbereich (20) das jeweilige Probenaufnahmeelement (14) hervortritt.

7. Gerät (2) nach Anspruch 6, **dadurch *gekennzeichnet,* daß** sich im Scheitelbereich (20) der Führung (8) eine Gleitkufe (42) befindet, durch welche das betreffende Probenaufnahmeelement (14) eine Auslenkung erfährt.

## Claims

1. Apparatus (2) for directed pick-up of biological samples (e.g. 46, 48) and transfer thereof, by means of a corresponding number of needle-like sample pick-up elements (14) within a support movable in a controlled manner, to positions of a flat sample receiving container, said positions being arranged along a line and at equal partition intervals, said sample pick-up elements being arranged in said support in regular partition intervals corresponding to those of the corresponding positions in said sample receiving container, **characterized in that** said support consists of a flexible strip (4) in which said sample pick-up elements (14) are arranged side by side, like the teeth of a comb, at partition intervals corresponding to those of the corresponding positions in said sample receiving container, and that said apparatus (2) further comprises a manually operable, hand-held switching and guiding device (6) for said strip (4), capable of removably receiving said strip and of indexing it in a stepwise manner in correspondence to said partition intervals of said sample pick-up elements in said strip and redirecting it in a manner that, in each index position of said strip, the tip of one of said sample pick-up elements (14) projects outwardly from the row of the remaining sample pick-up elements.

2. Apparatus (2) according to claim 1, **characterized in that** said sample pick-up elements (14) are formed laterally on said strip (4) and in the same plane.

3. Apparatus (2) according to claim 1 or 2, **characterized in that** said strip (4) comprises a transportation perforation (16) and said switching and guiding device (6) comprises a corresponding feed sprocket wheel (22).

4. Apparatus (2) according to the claims 2 and 3, **characterized in that** said strip (4) is made from a metal foil, preferably a V2A foil, by electrolytic etching.

5. Apparatus (2) according to claim 3, **characterized in that** said feed sprocket wheel (22) can be indexed by a manually operated stepping device (26) by individual partition angles corresponding to the partition intervals of said sample pick-up elements (14) in said strip 4).

6. Apparatus (2) according to any one of the preceding claims, **characterized in that** said switching and guiding device (6) comprises an approximately U-shaped guide (8) for said strip (4), the respective sample pick-up element (14) projecting from the apex region (20) thereof.

7. Apparatus (2) according to claim 6, **characterized in that**, in said apex region (20) of said guide (8), a slide shoe (42) is arranged by means of which the respective sample pick-up element (14) is deflected.

## Revendications

1. Appareil (2) destiné à une réception ciblée d'échantillons biologiques (par exemple 46, 48) et à leur transfert à des positions, disposées en une rangée et présentant des écarts de subdivision égaux, dans un récipient plat de réception d'échantillons, au moyen d'un nombre correspondant d'éléments de réception d'échantillons analogues à des aiguilles (14) à l'intérieur d'un support déplaçable de façon commandée, dans lequel ces éléments de réception d'échantillons sont agencés à des écarts de subdivision égaux correspondants à ceux des positions concernées dans le récipient de réception d'échantillons, **caractérisé en ce que** le support est constitué par une bande flexible (4) dans laquelle les éléments de réception d'échantillons (14) sont agencés les uns à côté des autres à la manière des dents d'un peigne, avec des écarts de subdivision correspondants à ceux des positions concernées dans le récipient de réception d'échantillons, et **en ce que** l'appareil (2) comporte en outre un dispositif de commutation et de guidage (6), actionné à la main et susceptible d'être arrêté à la main, pour la bande (4), ce dispositif convenant à recevoir la bande de façon amovible, et à faire progresser les éléments de réception d'échantillons pas à pas dans la bande en correspondance des écarts de subdivision, et à les dévier de telle manière que dans chaque position de commutation pas à pas de la bande, l'un des éléments de réception d'échantillons (14) respectif dépasse par sa pointe vers l'extérieur hors de la rangée des éléments de réception d'échantillons restants.

2. Appareil (2) selon la revendication 1, **caractérisé en ce que** les éléments de réception d'échantillons (14) sont réalisés à l'intérieur du même plan latéralement sur la bande (4).

3. Appareil (2) selon l'une ou l'autre des revendications 1 et 2, **caractérisé en ce que** la bande (4) comporte une perforation de transport (16), et ce que le dispositif de commutation et de guidage (6) comporte une roue dentée d'avance (22) adaptée à celle-ci.

4. Appareil (2) selon les revendications 2 et 3, **caractérisé en ce que** la bande (4) est fabriquée à partir d'une feuille métallique, de préférence une feuille V2A, et de préférence par attaque électrolytique à partir de cette feuille.

5. Appareil (2) selon la revendication 3, **caractérisé en ce que** la roue dentée d'avance (22) est susceptible d'être amenée à progresser au moyen d'un mécanisme de commutation pas à pas (26) à commande manuelle, d'un angle de subdivision respectif correspondant à l'écart de subdivision des éléments de réception d'échantillons (14) dans la bande (4).

6. Appareil (2) selon l'une des revendications précédentes, **caractérisé en ce que** le dispositif de commutation et de guidage (6) comporte un guidage (8) approximativement en forme de U pour la bande (4), et **en ce que** l'élément de réception d'échantillons respectif (14) dépasse dans la région de son sommet (20).

7. Appareil (2) selon la revendication 6, **caractérisé en ce que** dans la région du sommet (20) du guidage (8) se trouve un patin de coulissement (42) au moyen duquel l'élément de réception d'échantillons concerné (14) subit une déviation.
